# EUROPEAN PATENT APPLICATION

(11) **EP 2 510 929 A1**
(43) Date of publication of application: **17.10.2012**
(21) Application number: 11161898.9
(22) Date of filing: 11.04.2011
(51) Int. Cl.: A61K 31/5383, A61K 31/7036, A61K 45/06, A61K 38/39, A61P 17/02, A61P 31/02, A61P 31/04, A61L 26/00

(54) **Methods for treating bacterial infection**

(71) Applicant: Innocoll Technologies Limited, Roscommon (IE)
(72) Inventor: Prior, David, Dalkey, County Dublin (IE)
(74) Representative: O'Connell, Maura

(57) **Abstract**

This invention relates to methods for treating bacterial infection, which methods find utility in the treatment of, for example, infected ulcers, optionally infected diabetic ulcers. In particular, this invention relates to treating bacterial infection, for example, infected diabetic ulcers by topical administration of at least one aminoglycoside antibiotic at the site of infection, in combination with at least one antibacterial agent, which antibacterial agent is administered remote from the site of infection, preferably administered systemically. In a particular embodiment, the present invention relates to a composition for use in treating bacterial infection, the composition comprising gentamicin sulphate (a water-soluble broad-spectrum aminoglycoside antibiotic) uniformly dispersed in a type-I collagen matrix; in combination with at least one systemically-administered antibacterial agent. The present invention provides bactericidal activity against most strains of aerobic gram-negative and gram-positive and facultative anaerobic gram-negative pathogens, including methicillin-resistant *Staphylococcus aureus.*

## Description

This invention relates to methods for treating bacterial infection, which methods find utility in the treatment of, for example, infected ulcers, optionally infected diabetic ulcers. In particular, this invention relates to treating bacterial infection, for example, infected diabetic ulcers by topical administration of at least one aminoglycoside antibiotic at the site of infection, in combination with at least one antibacterial agent, which antibacterial agent is administered systemically.

### Background to the invention

Patients with diabetes are at high risk for developing foot ulcerations, which often become infected. These diabetic foot infections (DFIs) can cause substantial morbidity because of local pain and tissue destruction, as well as impaired wound healing, and sometimes lead to lower extremity amputations. The validated Infectious Diseases Society of America (IDSA) guideline for DFIs classifies their clinical severity as mild, moderate, or severe; depending on the size and depth of the infection and whether or not there are systemic manifestations or metabolic perturbations. The guideline's recommendations for selecting empirical antibiotic regimens are based on this classification. For mild infections, topical antimicrobials may be appropriate. One randomized controlled trial in patients with mild DFIs showed that treatment with a topical antimicrobial peptide (pexiganan) produced similar clinical outcomes to an oral antibiotic (ofloxacin). However, there are no topical antimicrobials approved for treating DFIs and their role in treatment of chronic wounds remains controversial. For most DFIs, systemic antibiotic therapy, either oral or parenteral, is prescribed. However, the need for better treatment approaches is highlighted by reported clinical failure rates of greater than 20%.

The gentamicin-sponge (Innocoll Technologies, Roscommon, Ireland) is an antibiotic delivery system for management of DFIs, composed of gentamicin sulphate (a water-soluble broad-spectrum aminoglycoside antibiotic) uniformly dispersed in a type-I collagen matrix. Gentamicin provides concentration-dependent bactericidal activity *in vitro* against most strains of aerobic gram-negative and gram-positive and facultative anaerobic gram-negative pathogens found in DFIs, including methicillin-resistant *Staphylococcus aureus.* Historically, there have been concerns that the topical application of gentamicin cream to skin ulcers may cause or propagate resistance to this agent, but one recent study of ocular isolates found no increased resistance to gentamicin when compared to other commonly used ocular antibiotics. Of note is that the creams and ointments indicated for skin infections contain only 0.1 % gentamicin; whereas, in one embodiment, the gentamicin sponge contains 27% gentamicin. This is intended to reduce the likelihood resistance propagation, as well as to increase its efficacy.

The gentamicin-sponge is approved in Europe as a biodegradable surgical implant for the adjuvant treatment of localized bone or soft tissue infections. Topical (as opposed to implantable) administration allows for repeated, rather than single, applications. This should theoretically maintain high concentrations of gentamicin in the wound environment over the course of treatment, while avoiding potentially serious toxicities associated with systemic administration.

### Summary of the invention

According to a first aspect of the present invention, there is provided a composition comprising at least one aminoglycoside antibiotic for use in treating bacterial infection, comprising:
(a) topical administration at the site of infection; and
(b) administration of at least one antibacterial agent, which antibacterial agent is administered remote from the site of infection.

According to a second aspect of the present invention, there is provided a method of treating bacterial infection at a site of infection, the method comprising the step of topically administering at least one aminoglycoside antibiotic at the site of infection; and administering at least one antibacterial agent, which at least one antibacterial agent is administered remote from the site of infection.

By "aminoglycoside antibiotic" is meant a compound comprising a polyatomic molecule comprising at least one sugar having at least one amino group substituent, and capable of providing a bacteriostatic or bactericidal effect.

Preferably, the at least one aminoglycoside antibiotic is gentamicin ((*3R,4R,5R*)-2-{[(1*S*,2*S*,3*R*,4*S*,6*R*)-4,6-diamino-3-{[(2*R*,3*R*,6*S*)-3-amino-6-[(1*R*)-1-(methylamino)ethyl]oxan-2-yl]oxy}-2-hydroxycyclohexyl]oxy}-5-methyl-4-(methylamino)oxane-3,5-diol), or a salt or prodrug thereof.

Optionally, the at least one aminoglycoside antibiotic is gentamicin sulphate. Further optionally, the gentamicin sulphate is administered in an equivalent amount of 32.5 - 130.0 mg of gentamicin.

Optionally, the at least one aminoglycoside antibiotic is administered topically as a composition comprising at least one aminoglycoside antibiotic and a polymeric matrix.

Optionally, the topical administering step comprises the step of topically applying a composition comprising at least one aminoglycoside antibiotic and a polymeric matrix to the site of infection.

Preferably, the at least one aminoglycoside antibiotic is uniformly (or homogenously) dispersed throughout the polymeric matrix.

Optionally, the polymeric matrix comprises collagen. Further optionally, the polymeric matrix comprises Type-I collagen.

Optionally, the at least one aminoglycoside antibiotic is uniformly (or homogenously) dispersed throughout the polymeric matrix in an amount of 0.1 - 10.0 mg/cm² of polymeric matrix. Further optionally, the at least one aminoglycoside antibiotic is uniformly (or homogenously) dispersed throughout the polymeric matrix in an amount of 1.0 - 5.5 mg/cm² of polymeric matrix. Still further optionally, the at least one aminoglycoside antibiotic is uniformly (or homogenously) dispersed throughout the polymeric matrix in an amount of 1.0 - 1.5 mg/cm² of polymeric matrix.

Optionally, the at least one aminoglycoside antibiotic is gentamicin sulphate and is uniformly (or homogenously) dispersed throughout the polymeric matrix in an amount equivalent to gentamicin of 1.3 mg/cm² of polymeric matrix.

Alternatively, the at least one aminoglycoside antibiotic is gentamicin sulphate and is uniformly (or homogenously) dispersed throughout the polymeric matrix in an amount equivalent to gentamicin of 5.2 mg/cm² of polymeric matrix.

For the purposes of this specification, the amount of the at least one aminoglycoside dispersed throughout the polymeric matrix refers to the amount of the at least one aminoglycoside antibiotic, which is uniformly (or homogenously) dispersed throughout the two-dimensional surface area of the polymeric matrix. It will be appreciated that the amount is exclusive of the three-dimensional surface area of the polymeric matrix. The two-dimensional surface area of the polymeric matrix refers to the two-dimensional surface area of the largest surface of the polymeric matrix and which, in use, contacts the site of infection.

Optionally, the at least one aminoglycoside antibiotic is gentamicin, or a salt or prodrug thereof, and is administered in an amount equivalent to gentamicin of 32.5 - 130.0 mg.

Optionally or additionally, the polymeric matrix has a two-dimensional surface area of 6.25 - 100 cm².

Optionally, the at least one aminoglycoside antibiotic is gentamicin, or a salt or prodrug thereof, and is administered at least once daily in an amount equivalent to gentamicin of 32.5 - 130.0 mg. Further optionally, the at least one aminoglycoside antibiotic is gentamicin, or a salt or prodrug thereof, and is administered at least twice daily in an amount equivalent to gentamicin of 32.5 - 130.0 mg. Further optionally, the at least one aminoglycoside antibiotic is gentamicin, or a salt or prodrug thereof, and is administered at least three times daily in an amount equivalent to gentamicin of 32.5 - 130.0 mg.

Optionally or additionally, the at least one aminoglycoside antibiotic is gentamicin, or a salt or prodrug thereof, and is administered, optionally at regular intervals, at least once weekly in an amount equivalent to gentamicin of 32.5 - 130.0 mg. Further optionally or additionally, the at least one aminoglycoside antibiotic is gentamicin, or a salt or prodrug thereof, and is administered, optionally at regular intervals, between one and six times weekly in an amount equivalent to gentamicin of 32.5 - 130.0 mg.

Optionally, the at least one aminoglycoside antibiotic is gentamicin, or a salt or prodrug thereof, and is administered topically as a composition comprising gentamicin, or a salt or prodrug thereof, in an amount equivalent to gentamicin of 32.5 - 130.0 mg; and a polymeric matrix.

The at least one antibacterial agent is administered systemically.

Optionally or additionally, the at least one antibacterial agent is administered by a route selected from oral and parenteral administration. Optionally or additionally, the at least one antibacterial agent is administered by parenteral administration selected from intravenous and intramuscular administration.

Optionally, the at least one antibacterial agent is selected from nitroimidazole compounds, lincosamides, sulfonamide compounds, dihydrofolate reductase inhibitors, lipopeptide molecules, tetracycline compounds, compounds comprising a beta-lactam moiety, glycopeptides, oxazolidinones, and quinolones.

Preferably, the nitroimidazole compound is a derivative of nitroimidazole (5-Nitro-1H-imidazole) and is capable of providing a bacteriostatic or bactericidal effect. Optionally, the nitroimidazole compound is metronidazole (2-(2-methyl-5-nitro-1*H*-imidazol-1-yl)ethanol).

Preferably, the lincosamide is a polyatomic molecule capable of providing a bacteriostatic or bactericidal effect. Optionally, the lincosamide is selected from lincomycin and clindamycin. Optionally, the lincosamide is clindamycin ((2*S*,4*R*)-*N*-{2-chloro-1-[(2*R*,3*R*,4*S*,5*R*,6*R*)-3,4,5-trihydroxy-6-(methylsulfanyl)oxan-2-yl]propyl}-1-methyl-4-propylpyrrolidine-2-carboxamide).

Preferably, the sulfonamide compound is a polyatomic molecule comprising a sulfonamide functional group (-S(=O)₂-NH₂) and is capable of providing a bacteriostatic or bactericidal effect. Optionally, the sulfonamide compound is sulfamethoxazole (4-amino-*N*-(5-methylisoxazol-3-yl)-benzenesulfonamide).

Optionally, the sulfonamide compound is administered with a dihydrofolate reductase inhibitor.

Preferably, the dihydrofolate reductase inhibitor is a molecule capable of inhibiting the biological function of dihydrofolate reductase and is capable of providing a bacteriostatic or bactericidal effect. Optionally, the dihydrofolate reductase inhibitor is trimethoprim (5-(3,4,5- trimethoxybenzyl) pyrimidine- 2,4- diamine).

Preferably, the lipopeptide molecule comprises a lipid moiety and a polypeptide moiety and is capable of providing a bacteriostatic or bactericidal effect. Optionally, the lipopeptide molecule is daptomycin (*N*-decanoyl-L-tryptophyl-L-asparaginyl-L-aspartyl-L-threonylglycyl-L-ornithyl-L-aspartyl-D-alanyl-L-aspartylglycyl-D-seryl-*threo* -3-methyl-L-glutamyl-3-anthraniloyl-L-alanine[egr]₁-lactone).

Preferably, the tetracycline compound is a polyatomic molecule capable of providing a bacteriostatic or bactericidal effect. Optionally, the tetracycline compound is doxycycline ((4*S*,4a*R*,5*S*,5a*R*,6*R*,12a*S*)-4-(dimethylamino)- 3,5,10,12,12a-pentahydroxy- 6-methyl- 1,11-dioxo-1,4,4a,5,5a,6,11,12a-octahydrotetracene- 2-carboxamide).

Alternatively, the tetracycline compound is a derivative of tetracycline (*N*-[(5a*R*,6aS,7*S*,9*Z*,10a*S*)-9-[amino(hydroxy)methylidene]-4,7-*bis*(dimethylamino)-1,10a,12-trihydroxy-8,10,11-trioxo-5,5a,6,6a,7,8,9,10,10a, 11-decahydrotetracen-2-yl]-2-(*tert*-butylamino)acetamide).

Optionally, the tetracycline compound is a glycylcycline compound. Further optionally, the tetracycline compound is tigecycline (*N*-[(5a*R*,6a*S*,7*S*,9*Z*,10a*S*)-9-[amino(hydroxy)methylidene]-4,7-*bis*(dimethylamino)-1,10a,12-trihydroxy-8,10,11-trioxo-5,5a,6,6a,7,8,9,10,10a,11-decahydrotetracen-2-yl]-2-(*tert*-butylamino)acetamide).

Preferably, the compound comprising a beta-lactam moiety is a polyatomic molecule capable of providing a bacteriostatic or bactericidal effect. Optionally, the compound comprising a beta-lactam moiety is selected from cephalosporins, carbapenems, penicillins, and aztreonam.

Optionally, the cephalosporin is selected from cefazolin, cefalexin, cefalotin, cefdinir, cefepime, cefotaxime, cefpodoxime proxetil, ceftobiprole, and ceftaroline fosamil.

Optionally, the carbapenem is selected from ertapenem ((4*R*,5*S*,6*S*)-3-[(3*S*,5*S*)-5-[(3-carboxyphenyl)carbamoyl]pyrrolidin-3-yl]sulfanyl-6-(1-hydroxyethyl)-4-methyl-7-oxo-1-azabicyclo[3.2.0]hept-2-ene-2-carboxylic acid) and meropenem (3-[5-(dimethylcarbamoyl) pyrrolidin-2-yl] sulfanyl-6- (1-hydroxyethyl)-4-methyl-7-oxo- 1-azabicyclo[3.2.0] hept-2-ene-2-carboxylic acid).

Optionally, the penicillin is selected from amoxicillin, ampicillin, methicillin, oxacillin, nafcillin, cloxacillin, dicloxacillin, and flucloxacillin.

Optionally or additionally, the penicillin is administered with a beta-lactamase inhibitor.

Optionally or additionally, the amoxicillin is administered with the beta-lactamase inhibitor clavulanic acid ((2*R*,5*R*,Z)- 3-(2-hydroxyethylidene)- 7-oxo- 4-oxa- 1-aza- bicyclo[3.2.0]heptane- 2-carboxylic acid).

Optionally or additionally, the ampicillin is administered with the beta-lactamase inhibitor sulbactam ((2*S*,*5*R)-3,3-dimethyl-7-oxo-4-thia-1-azabicyclo[3.2.0]heptane-2-carboxylic acid 4,4-dioxide).

Optionally, the penicillin is selected from dicloxacillin ((2*S*,5*R*,6*R*)-6-{[3-(2,6-dichlorophenyl)-5-methyl-oxazole-4-carbonyl]amino}-3,3-dimethyl-7-oxo-4-thia-1-azabicyclo[3.2.0]heptane-2-carboxylic acid), flucloxacillin ((2*S*,5*R*,6*R*)-6-({[3-(2-chloro-6-fluorophenyl)-5-methylisoxazole-4-yl]carbonyl}amino)-3,3-dimethyl-7-oxo-4-thia-1-azabicyclo[3.2.0]heptane-2-carboxylic acid), and oxacillin ((2*S*,5*R*,6*R*)-3,3-dimethyl-6-[(5-methyl-3-phenyl-1,2-oxazole-4-carbonyl)amino]-7-oxo-4-thia-1-azabicyclo[3.2.0]heptane-2-carboxylic acid); preferably selected from dicloxacillin and flucloxacillin.

Alternatively, the penicillin is selected from azlocillin, carbenicillin, ticarcillin, mezlocillin, and piperacillin.

Preferably, the penicillin is piperacillin ((2*S*,5*R*,6*R*)-6-{[(2*R*)-2-[(4-ethyl-2,3-dioxo-piperazine-1-carbonyl)amino]-2-phenyl-acetyl]amino}-3,3-dimethyl-7-oxo-4-thia-1-azabicyclo[3.2.0]heptane-2-carboxylic acid).

Optionally or additionally, the piperacillin is administered with the beta-lactamase inhibitor tazobactam ((2*S*,3*S*,5*R*)-3-methyl-7-oxo-3-(1*H*-1,2,3-triazol-1-ylmethyl)-4-thia-1-azabicyclo[3.2.0]heptane-2-carboxylic acid 4,4-dioxide).

Alternatively, the compound comprising a beta-lactam moiety is aztreonam.

By "glycopeptides" is meant a compound comprising a polyatomic molecule comprising glycosylated or polycyclic non-ribosomal peptides, and capable of providing a bacteriostatic or bactericidal effect.

Optionally, the glycopeptide is selected from dalbavancin, vancomycin, teicoplanin, talavancin, and bleomycin.

Preferably, the glycopeptide is vancomycin ((1*S*,2*R*,18*R*,19*R*,22*S*,25*R*,28*R*,40*S*)- 48-{[(2*S*,3*R*,4*S*,5*S*,6*R*)- 3- {[(2*S*,4*S*,5*S*,6*S*)- 4- amino- 5- hydroxy- 4,6- dimethyloxan- 2- yl]oxy}- 4,5-dihydroxy- 6- (hydroxymethyl)oxan- 2- yl]oxy}- 22- (carbamoylmethyl)- 5,15- dichloro- 2,18,32,35,37-pentahydroxy- 19-[(2*R*)- 4- methyl- 2- (methylamino)pentanamido]- 20,23,26,42,44- pentaoxo- 7,13-dioxa- 21,24,27,41,43- pentaazaoctacyclo[26.14.2.2^{3,6}.2^{14,17}.1^{8,12}.1^{29,33}.0^{10,25}.0^{34,39}]pentaconta-3,5,8(48),9,11,14,16,29(45),30,32,34,36,38,46,49- pentadecaene- 40- carboxylic acid).

By "oxazolidinone" is meant a heterocyclic compound comprising a nitrogen atom, and oxygen atom in a 5-membered ring, and capable of providing a bacteriostatic or bactericidal effect.

Optionally, the oxazolidinone is linezolid ((*S*)-*N*-({3-[3-fluoro-4-(morpholin-4-yl)phenyl]-2-oxo-1,3-oxazolidin-5-yl}methyl)acetamide).

Optionally, the at least one antibacterial agent is selected from fluoroquinolones.

By "fluoroquinolone" is meant a compound comprising a polyatomic molecule comprising at least one quinolone moiety having at least one fluorine substituent, and capable of providing a bacteriostatic or bactericidal effect.

Optionally, the fluoroquinolone is selected from ciprofloxacin, moxifloxacin, ofloxacin, balofloxacin, grepafloxacin, levofloxacin, pazufloxacin, sparfloxacin, temafloxacin, and tosufloxacin. Further optionally, the fluoroquinolone is selected from ciprofloxacin, moxifloxacin, and levofloxacin.

Preferably, the fluoroquinolone is levofloxacin ((*S*)-7-fluoro-6-(4-methylpiperazin-1-yl) -10-oxo-4-thia-1-azatricyclo[7.3.1.0^{5,13}] trideca-5(13),6,8,11-tetraene-11-carboxylic acid).

Optionally, the at least one aminoglycoside antibiotic is co-administered in combination with the at least one antibacterial agent.

Optionally or additionally, the at least one antibacterial agent is administered prior to, and is co-administered in combination with the at least one aminoglycoside antibiotic. The at least one antibacterial agent can be administered for a period of 1-3 days prior to, and is co-administered in combination with the at least one aminoglycoside antibiotic.

Optionally, the bacterial infection is an infection by a bacteria selected from at least one of an aerobic gram-negative bacteria, aerobic gram-positive bacteria, and anaerobic gram-negative bacteria.

The bacterial infection may comprise more than one of an aerobic gram-negative bacteria, aerobic gram-positive bacteria, and anaerobic gram-negative bacteria.

Optionally, the gram-positive bacteria is selected from *Streptococcus, Staphylococcus, Enterococcus,* Gram positive cocci, and *Peptostreptococcus.*

Further optionally, the gram-positive bacteria is selected from beta-hemolytic *Streptococcus,* coagulase negative *Staphylococcus, Enterococcus faecalis* (VSE), *Staphylococcus* aureus, and *Streptococcus pyogenes.*

Still further optionally, the gram-positive bacteria is selected from methicillin-sensitive *Staphylococcus* aureus (MSSA), and methicillin-resistant *Staphylococcus aureus* (MRSA).

Preferably, in the case of a gram-positive bacterial infection, the at least one antibacterial agent is selected from lincosamides, sulfonamide compounds, dihydrofolate reductase inhibitors, lipopeptide molecules, tetracycline compounds, quinolones, oxazolidinones, glycopeptides, and compounds comprising a beta-lactam moiety. Further preferably, in the case of a gram-positive bacterial infection, the at least one antibacterial agent is selected from clindamycin, sulfamethoxazole administered with trimethoprim, daptomycin, doxycycline, tigecycline, ertapenem, meropenem, amoxicillin administered with clavulanic acid, ampicillin administered with sulbactam, dalbavancin, ciprofloxacin, moxifloxacin, ofloxacin, levofloacin, linezolid, vancomycin, dicloxacillin, flucloxacillin, and oxacillin.

Optionally, the gram-negative bacteria is selected from *Acinetobacter, Alcaligenes, Bacteroides, Burkholderia, Enterobacter, Klebsiella, Morganella, Ochrobactrum, Proteus, Providencia, Pseudomonas,* and *Serratia.*

Further optionally, the gram-negative bacteria is selected from *Alcaligenes faecalis, Bacteroides fragilis, Enterobacter cloacae, Klebsiella oxytoca, Morganella morganii, Ochrobactrum anthropi, Providencia rettgeri, Pseudomonas aeruginosa,* and *Serratia marcescens.*

Preferably, in the case of a gram-negative bacterial infection, the at least one antibacterial agent is selected from nitroimidazole compounds, sulfonamide compounds, dihydrofolate reductase inhibitors, tetracycline compounds, quinolones, and compounds comprising a beta-lactam moiety. Further preferably, in the case of a gram-negative bacterial infection, the at least one antibacterial agent is selected from metronidazole, sulfamethoxazole administered with trimethoprim, doxycycline, tigecycline, ertapenem, meropenem, amoxicillin administered with clavulanic acid, ampicillin administered with sulbactam, ciprofloxacin, moxifloxacin, ofloxacin, levofloxacin, piperacillin, piperacillin administered with tazobactam, and aztreonam.

Optionally, the bacterial infection is selected from a soft tissue bacterial infection, a hard tissue bacterial infection, or a combination thereof.

Optionally, the bacterial infection is a hard tissue bacterial infection, for example, osteomyelitis.

According to a third aspect of the present invention, there is provided a composition comprising at least one aminoglycoside antibiotic for use in treating infected ulcers, optionally infected diabetic ulcers, comprising:
(a) topical administration at the site of infection; and
(b) administration of at least one antibacterial agent, which antibacterial agent is administered remote from the site of infection.

According to a fourth aspect of the present invention, there is provided a method of treating infected ulcers, optionally infected diabetic ulcers, the method comprising the step of topically administering at least one aminoglycoside antibiotic at the site of infection; and administering at least one antibacterial agent, which at least one antibacterial agent is administered remote from the site of infection.

Optionally, the diabetic ulcer is a diabetic lower limb ulcer. Further optionally, the diabetic ulcer is a diabetic foot ulcer.

Optionally, the infection is a bacterial infection. Optionally, the bacterial infection is the bacterial infection of a wound.

Optionally, the wound is selected from venous stasis ulcers, arterial ulcers, decubitus ulcers, surgical wounds, radiation ulcers, and wounds caused by a burn.

The infected diabetic ulcer has a clinical severity selected from mild, moderate, and severe, according to the validated Infectious Diseases Society of America (IDSA) guideline. Optionally, the diabetic ulcer has a moderate or severe clinical severity, according to the validated Infectious Diseases Society of America (IDSA) guideline.

### Brief description of the drawings

Figure 1 is a graph depicting the probability of achieving baseline pathogen eradication;
Figure 2 is a graph depicting serum gentamicin concentrations; and
Figure 3 is a flow-chart depicting the disposition of all enrolled patients.

### Method of manufacture of a polymeric collagen matrix comprising gentamicin

### Collagen extraction and purification

Collagen can be extracted from a number of sources including animal hides and animal tendons. The extraction process for collagen was according to standard practice and well known to those skilled in the art. In short, during the manufacturing process, milled bovine tendons were treated with a number of reagents, for example, with 1 N sodium hydroxide (NaOH) to remove microbiological contamination, including prions.

Further reduction of the particle size of the collagen-containing material was followed by treatment with pepsin at approximate pH of 2.5 to degrade contaminating serum components, primarily bovine serum albumin and to cause detachment of non-helical portions of the collagen molecule (telopeptides). During this process, the collagen material was partially solubilised in the acidic medium. After filtration, precipitation of the collagen was accomplished by means of manipulation of the pH (from pH of about 2.5 to pH of about 7.5). The fibrillar collagen material was finally precipitated out of solution and then concentrated by means of centrifugation.

### Compounding process and equipment

A collagen dispersion was manufactured in a stainless steel vessel. The aqueous-based dispersion was prepared using pre-heated (38-42°C) water and adjusted to pH 4.5±0.2. Without being bound by theory, it is thought that, when the gentamicin sulphate is added to the collagen dispersion at pH 4.5, the collagen sediments out and to reverse this, the pH is dropped in the range of 3.5 to 4.0 to allow the collagen to become re-dispersed (fibres are more swollen and solubility increases at the lower pH). High shear mixing was required to break up the collagen mass and expose the collagen fibres to the acidic medium. The homogeniser employed possessed a rotor-stator head that is designed to create high shear forces by pulling the material through the rotating homogeniser head and forcing it against the proximal stationary stator head. It was this design that facilitates the separation of the fibrous collagen mass at the beginning of dispersion preparation.

The rotor/stator equipment used in the manufacturing process was selected based on existing in-house experience with this equipment. For example, an IKA Ultra-Turrax mixer can be used at a high speed for about 1-10 minutes, followed by low shear mixing after the addition of the drug for a minimum of 20 minutes.

Following completion of collagen dispersion formation, the dispersion was transferred to a closed heat-jacketed stainless steel vessel for final compounding. The jacket temperature was maintained at 40 ± 2 °C. Gentamicin sulphate [Fujian Fukang Pharmaceutical Co., Ltd., Fujian, China] was first dissolved in a portion of acidified water under manual stirring; this solution premix was then introduced into the heat-jacketed SS vessel under low shear mixing to achieve homogeneity in the drug-loaded collagen dispersion. The final drug/collagen dispersion was adjusted to pH 4.5±0.2 prior to filling into lyophilisation moulds and the collagen concentration was 0.56g/100g.

### Filling/lyophilisation process and equipment

The filling process was performed using a positive displacement pump. The pump was valve-less, had ceramic or stainless steel pistons and worked on the principle of positive displacement. Alternatively, a peristaltic pump can be used. To produce a 5x5 cm unit, 12.5 grams of the drug-collagen dispersion (containing the equivalent of 50 mg gentamicin sulphate) was filled into a 5x5 cm mould and to produce a 10x10 cm unit, 50 grams (containing the equivalent of 200 mg gentamicin sulphate) was filled into a 10x10 cm mould. Upon completion of filling, the filled moulds were placed into a lyophiliser. A commercially available lyophiliser (e.g. Christ lyophiliser) was utilised for lyophilisation of the gentamicin-containing sponges. The lyophiliser-operating console permitted process cycle programming and an automated program cycle was established for the product to yield the lyophilised sponge with approximate dimensions of 5x5 cm or 10x10 cm (according to the inner dimensions of the mould), with a thickness in the range of about 3.5 to about 5.0mm.

### Packaging process and equipment

Following completion of the lyophilisation cycle, the trays were removed from the shelves. The resultant gentamicin-collagen sponges were removed from the moulds and packed into pouches. The packaging process proceeded in two steps, inner pouch packaging and sealing; and outer pouch packaging and sealing (both suitable for gaseous ethylene oxide [EO] sterilization). The sponge product was placed into an inner pouch and sealed with an impulse-sealing machine. A continuous sealer can also be used. One side of the inner pouch comprised transparent polyester/LDPE foil laminate, the other side comprised DuPont™ Tyvek®, although other suitable materials can be selected by one skilled in the art. The sealed inner pouch was then inserted into an outer EO sterilisable pouch. One side of the outer pouch comprised a transparent PETP/LDPE foil laminate with a DuPont™ Tyvek® strip seal while the other side was an opaque PETP/PE laminate. Other outer pouch packaging can be used including aluminium oxide coated polyethylene materials or if electron (E) beam radiation is used for sterilisation, an aluminium outer pouch can be used. The pouch was then sealed using continuous heat-sealing equipment. The heat sealer facilitated the formation of a continuous seal at the open end of the pouch. The top part of the pouch included two holes or a strip lined with DuPont™ Tyvek®. These holes with the DuPont™ Tyvek® material are specifically designed for the EO gas sterilisation process and are gas permeable only. The permeability of the holes facilitates permeation of EO gas during the terminal sterilisation process. The sealed product was transferred to the terminal EO sterilisation process stage. Following sterilisation and ventilation, the outer pouch was resealed below the gas permeable window and this gas permeable (top) portion was then removed from the pouch. This resulted in a fully sealed outer pouch containing a terminally sterilised sponge product.

### Experimental design and methods

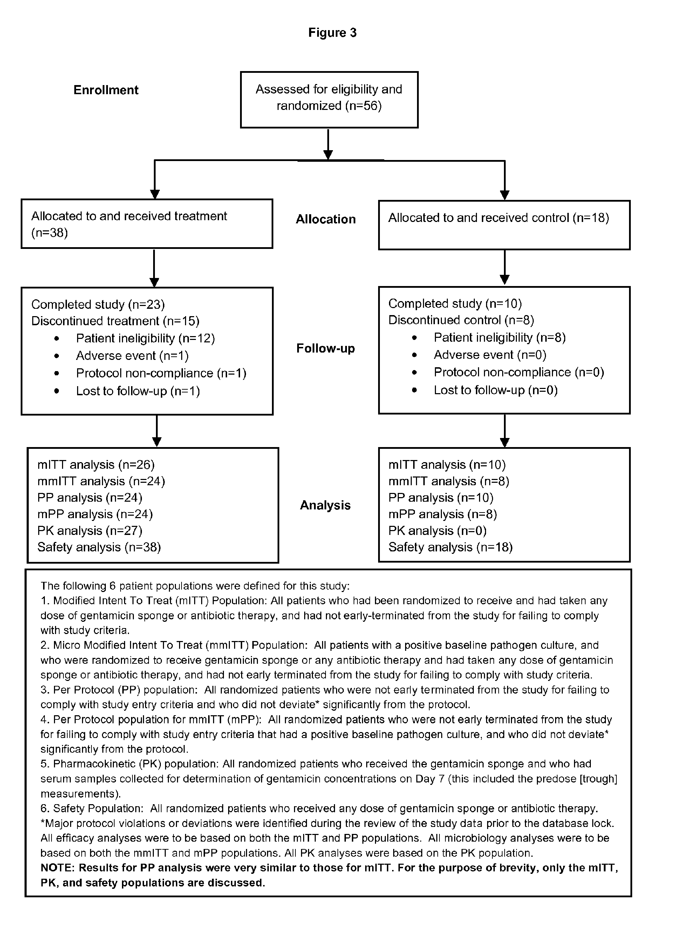

Eligible subjects for the present randomized, controlled, open-label, multicenter pilot study were diabetic patients aged 18-80 years with a single, moderately infected lower extremity ulcer. A moderately infected ulcer was defined by the IDSA guideline criteria, i.e., a patient who is systemically well and metabolically stable with a foot wound showing ≥2 manifestations of inflammation (purulence or erythema, pain, tenderness, warmth or induration) and ≥1 of the following characteristics: cellulitis extending 2 cm, lymphangitic streaking, spread beneath the superficial fascia, deep tissue abscess, gangrene, or involvement of muscle, tendon, joint or bone. The ulcers were graded using the Lipsky Wound Scoring Scale, and patients with an ulcer that could not be completely covered with a single gentamicin sponge were excluded. Patients who had received any antimicrobial therapy in the preceding 2 weeks, as well as those with ischemia of the lower limb (defined as an ankle-brachial index < 0.7 or > 1.3 unless they had either a transcutaneous oxygen pressure or toe pressure ≥ 40 mm Hg) and, at Institutional Review Board (IRB) request, patients with a glycated hemoglobin (HbA1c) level of ≥10.0% were also excluded. See Figure 3.

This study was designed to compare the effect on the clinical outcome of infection of treatment with daily application of the gentamicin-sponge (either 5×5 cm in size with 50 mg gentamicin sulfate [equivalent to 32.5 mg gentamicin base] or 10×10 cm with 200 mg gentamicin sulfate [equivalent to 130 mg gentamicin base]) in combination systemic antibiotic therapy (daily oral or intravenous [IV] dose of 750 mg levofloxacin or alternative antimicrobial therapy, as determined by susceptibility testing, plus standard diabetic wound management) to systemic antibiotic therapy alone. Levofloxacin was selected as the first choice systemic antibiotic based upon its proven efficacy in complicated skin and soft tissue (including diabetic foot) infections, its listing among the recommended agents in the IDSA Guidelines, and the convenience of its once-daily dosing. Patients in both arms also received standard diabetic wound management, including sharp surgical debridement at each visit where appropriate, pressure off-loading as applicable and daily dressing changes using a nonadherent, moisture permeable dressing followed by a secondary saline-moistened gauze dressing. To keep the dressings in place, roll gauze was wrapped circumferentially around the wound and secured with nonirritating tape.

It was chosen not to administer placebo collagen sponges to control patients because of a concern that a placebo sponge could potentially harbour bacteria and bias the results in favour of the active treatment. Therefore, to reduce the complexity of this pilot study, an open-label design was chosen. Patients were provided with levofloxacin tablets, an off-loading device, dressings for standard wound management, and gentamicin-sponges, as necessary, for daily outpatient treatment between study visits. Patients or their caregivers were also instructed in the use of the off-loading device and method of dressing change, to include dressing removal and wound irrigation with sterile saline. Patients were treated for at least 7 days and continued treatment until the investigator determined that all signs and symptoms of infection had resolved, to a maximum of 28 days. The test-of-cure and safety assessment was scheduled 2 weeks after discontinuation of treatment, for a total study duration of up to 42 days.

Patients at 8 sites in the United States and 1 site in the United Kingdom were enrolled in the study and underwent screening procedures, including routine laboratory tests and culture of a tissue specimen of their ulcer, at their first visit. Patients who were deemed eligible provided written informed consent and were randomized in a 2:1 ratio to the treatment or control group using an interactive voice response system. Randomization was not stratified by any baseline characteristic. Enrolled subjects all received 750 mg oral levofloxacin empirically, while awaiting the results of wound culture and susceptibility testing. For subjects who were randomized to receive the gentamicin-sponge, the sponge size to apply was determined so that the wound was completely covered (10×10 cm sponge for ulcers with a maximum length >5 cm or a 5×5 cm sponge for ulcers ≤5 cm in length). When baseline laboratory results were available, subjects who were no longer deemed eligible for the study due to an out of range laboratory value or a levofloxacin-resistant isolate were withdrawn from the study.

Clinical and microbiological assessments were performed at regular study visits while the patient was on antibiotic therapy (i.e., Days 3, 7, 10, 14, 21, and 28) and again at the follow-up visit. Clinical outcomes were defined as follows:
- Clinical Cure: Resolution of all baseline signs and symptoms of infection.
- Clinical Improvement: Improvement of ≥1, but not all, of the baseline signs and symptoms of infection.
- Failure: No improvement of any of the baseline signs and symptoms of infection.

A third party blinded to the treatment regimen determined microbiological outcomes by isolated pathogen. These were classified as: "documented eradication," "presumed eradication," "documented persistence," "presumed persistence," or "unknown". A by-patient outcome of "microbiological success" or "microbiological failure" was also assigned, based upon culture results obtained at the final visit or early termination.

Minimum inhibitory concentration (MIC) testing was performed on all isolated pathogens to determine susceptibility to systemic concentrations of levofloxacin, linezolid, vancomycin and oxacillin for gram-positive species and levofloxacin, aztreonam and piperacillin/tazobactam for gram-negative species. Definitive gentamicin MIC testing against all isolated pathogens up to 1024 µg/mL to mimic high local concentrations were also performed.

After wound debridement, the ulcer was graded using the Lipsky Wound Score at baseline and all subsequent visits. Additionally, study personnel who were blinded to the treatment regimen obtained wound tracings. A centralized imaging facility (Canfield Scientific, Fairfield, NJ) calculated the wound surface area from the tracings using a validated custom application based on Image Pro Plus (Media Cybernetics, Bethesda, MD).

For all patients who received the gentamicin-sponge, pre-dose trough pharmacokinetic samples were collected on day 7 within the 30 minutes before sponge application to determine if there was any systemic accumulation of gentamicin from the earlier applications. Serum gentamicin concentrations were also measured for up to 8 hours following sponge application on day 7 for all 5 patients who received the 10×10 cm sponge, and for the first 3 patients who received the 5×5 cm sponge.

Safety data were collected at scheduled intervals during the study, including physical findings, vital signs and laboratory assessments and recorded all adverse events (AEs). Using standard definitions, an AE was any clinically unfavourable and unintended sign (including abnormal laboratory findings), symptom or disease, temporally associated with the treatment, whether or not it was causally related to the treatment. A serious adverse event (SAE) was any AE that resulted in death, was life-threatening, required inpatient hospitalization or prolongation of existing hospitalization, resulted in permanent disability/incapacity, caused a congenital abnormality or was an important medical event. Each AE was designated based on its clinical severity as either "mild" (caused no limitation of usual activities), "moderate" (caused some limitation of usual activities), or "severe" (prevented or severely limited usual activities). Its relationship to treatment was assessed as either "definitely related", "probably related", "unlikely related", or "not related."

To improve patient enrollment, several amendments to the original protocol were made, including widening the patient age range, decreasing the number of patients requiring post-dose pharmacokinetic sampling, and reducing the duration of post-dose sampling. Additionally, the exclusion criteria "history of epilepsy" and "tendon disorders related to fluoroquinolone administration" were added, which was necessitated by a new levofloxacin "black box" warning.

### End points and statistical methods

The primary efficacy endpoint for this study was the percent of patients with a clinical outcome of "clinical cure" at day 7. Assuming 70% of treatment patients and 40% of control patients would achieve this outcome, it was calculated that a sample size of 50 treatment and 25 control patients was needed to achieve 70% power to detect a difference between the two groups at the P = 0.05 significance level using a 2-sided chi-square test, and a 2:1 randomization ratio. Statistical calculations suggested that a substantially larger sample size was needed to test for treatment superiority at the test-of-cure visit, given the higher rates of clinical cure expected. Therefore, the day 7 visit was selected as the primary endpoint to satisfy an appropriate sample size for this study.

Secondary efficacy endpoints included the following: percent of patients with clinical cure at all time points other than day 7; percent with a positive clinical response (defined as clinical cure or clinical improvement); percent with pathogen eradication at each time point (each of which were summarized descriptively and compared across treatments using the chi-square test); change in Lipsky Wound Score; and, total wound surface area (which were compared across treatments using 2-sample t-tests); time to clinical cure; time to positive clinical response; and, time to eradication of baseline pathogens (which were calculated for each patient and summarized using the Kaplan-Meier method with log-rank tests used to compare treatment groups). Antibiotic susceptibility testing of isolated pathogens was summarised descriptively by the specific antibiotic agent. For patients with post-dose pharmacokinetic assessments, the individual gentamicin serum concentration time profiles and corresponding mean profiles for each sponge size were plotted. Safety evaluations included summary reports for the incidence and severity of all AEs, as described above.

### Ethics

This study was approved by either Quorum Review IRB, Seattle WA or a review board at the trial centre, and the studies were conducted in accordance with the Declaration of Helsinki and Good Clinical Practice guidelines.

### Examples

Embodiments of the present invention will now be described by way of accompanying non-limiting examples.

### Example 1 - Patient Disposition

From April 2008 to the last patient's final visit in May 2009, a total of 56 patients were enrolled; 38 were randomized to the treatment group and 18 to the control group. It was decided to stop the trial before reaching the targeted sample size because enrolment in this study was more difficult than anticipated - this was largely related to the inclusion/exclusion criteria being too restrictive, particularly the requirements that patients have only a single ulcer and not have received any systemic or topical antimicrobial therapy in the preceding 2 weeks. It was also found that many randomized patients were subsequently deemed ineligible once baseline laboratory results became available.

The disposition of all enrolled patients is summarized in Figure 3. Of 56 patients randomized, 33 (59%) completed the study while 23 were discontinued prematurely. The most common reason (20/23) for discontinuation was ineligibility because the patient failed to meet all of the inclusion and exclusion criteria. One (1) patient was deemed ineligible on the day of randomization for having an underlying medical condition that would interfere with result interpretation and 19 patients (11 in the treatment group and 8 in the control group) were deemed ineligible once baseline laboratory results became available; 14 due to excessively high HbA1c, one of whom also had elevated AST and ALT levels, 1 due to high serum creatinine, 1 due to high ALT, and, 3 because of levofloxacin-resistant isolates. Because such a high proportion (i.e., 36%) of randomized patients were deemed ineligible, a modified intention-to-treat (mITT) population was used to exclude these patients from all efficacy analyses.

The 3 eligible patients in the mITT who did not complete the study were all randomized to the treatment group. One clinically-improved patient was withdrawn at day 9 due to a SAE (hypoglycemia) unrelated to the gentamicin-sponge, another was clinically cured by day 14 but was hospitalized before the follow-up visit because of tendon rupture attributed to oral levofloxacin (officially "lost to follow-up"), and the third was withdrawn for failing to apply the sponges (protocol noncompliance).

### Example 2 - Patient Demographic Characteristics

Demographic characteristics of enrolled patients were not significantly different between the groups (See Table 1). However, assessment of baseline characteristics in the mITT population revealed an unfortunate imbalance in baseline wound severity. Baseline Lipsky Wound Scores were significantly higher in the gentamicin-sponge than the control group (median 17 vs. 12, P = 0.011) and the mean baseline wound surface area was almost 4-fold larger for patients randomized to the treatment group (5.11 cm²) than the control group (1.24 cm²).

**Table 1. Patient Demographic Characteristics**

| **Parameter** | **Group** | |
|---|---|---|
| | **Treatment (N=38)** | **Control (N=18)** |
| **Age (years)** | | |
| Mean (SD) | 57.9 (11.47) | 54.7 (12.80) |
| Median (minimum, maximum) | 58.0 (24, 80) | 54.5 (29, 81) |
| **Gender, n (%)** | | |
| Male | 23 (60.5%) | 15 (83.3%) |
| Female | 15 (39.5%) | 3 (16.7%) |
| **Race^{a}, n (%)** | | |
| American-Indian or Alaskan Native | 1 (2.6%) | 0 (0%) |
| Black or African American | 4 (10.5%) | 3 (16.7%) |
| Native Hawaiian or Other Pacific Islander | 1 (2.6%) | 0 (0%) |
| White or Caucasian | 32 (84.2%) | 15 (83.3%) |
| **Ethnicity, n (%)** | | |
| Hispanic or Latino | 12 (31.6%) | 5 (27.8%) |
| Not Hispanic or Latino | 26 (68.4%) | 13 (72.2%) |
| BMI (kg/m²) | | |
| Mean (SD) | 32.38 (6.500)^{b} | 32.67 (5.796) |
| Median (minimum, maximum) | 32.30 (21.1, 44.8)^{b} | 31.70 (23.7, 45.1) |

| | | |
|---|---|---|
| ^{a} Counts for race may not sum to total as multiple responses per patient were possible. ^{b} Mean and median BMI in treatment group based on measurements for 37 patients. | | |

### Example 3 - Isolated pathogens

Pathogens isolated from wounds at baseline are summarized in Table 2. The most commonly identified baseline pathogen for both treatment groups was *Staphylococcus aureus,* about equally divided between methicillin-resistant (MRSA) and methicillin-sensitive (MSSA) species. No patient was switched from oral levofloxacin to an alternative antibiotic regimen on the basis of the culture and antibiotic susceptibility results.

**Table 2. Pathogens Isolated From Diabetic Foot Wounds at Baseline**

| | **mITT Population^{a}** | | **Safety Population^{a}** | |
|---|---|---|---|---|
| | **Treatment (N=26)** | **Control (N=10)** | **Treatment (N=38)** | **Control (N=18)** |
| **Gram positive** | | | | |
| β -hemolytic | 5 (19.2) | 2 (20.0) | 7 (18.4) | 3 (16.7) |
| *Streptococcus* | | | | |
| Coagulase-negative | 7 (26.9) | 0 (0.0) | 10 (26.3) | 3 (16.7) |
| *Staphylococcus* spp | | | | |
| *Enterococcus* | 2 (7.7) | 4 (40.0) | 4 (10.5) | 5 (27.8) |
| *faecalis* (VSE) | | | | |
| Gram positive cocci | 0 (0.0) | 0 (0.0) | 1 (2.6) | 0 (0.0) |
| (NOS) | | | | |
| *Peptostreptococcus* | 1 (3.8) | 1 (10.0) | 2 (5.3) | 1 (5.6) |
| spp. | | | | |
| *Staphylococcus* | 7 (26.9) | 5 (50.0) | 9 (23.7) | 5 (27.8) |
| *aureus* (MRSA) | | | | |
| *Staphylococcus* | 6 (23.1) | 3 (30.0) | 10 (26.3) | 5 (27.8) |
| *aureus* (MSSA) | | | | |
| *Streptococcus* | 0 (0.0) | 1 (10.0) | 0 (0.0) | 1 (5.6) |
| *pyogenes* | | | | |
| **Gram negative** | | | | |
| *Acinetobacter* Sp. | 1 (3.8) | 0 (0.0) | 1 (2.6) | 2 (11.1) |
| *Alcaligenes faecalis* | 1 (3.8) | 0 (0.0) | 1 (2.6) | 0 (0.0) |
| *Bacteroides fragilis* | 0 (0.0) | 1 (10.0) | 1 (2.6) | 1 (5.6) |
| *Burkholderia* Sp. | 1 (3.8) | 0 (0.0) | 1 (2.6) | 0 (0.0) |
| *Enterobacter* | 1 (3.8) | 0 (0.0) | 1 (2.6) | 1 (5.6) |
| *cloacae* | | | | |
| *Klebsiella oxytoca* | 3 (11.5) | 0 (0.0) | 3 (7.9) | 2 (11.1) |
| *Morganella* | 0 (0.0) | 0 (0.0) | 0 (0.0) | 1 (5.6) |
| *morganii* | | | | |
| *Ochrobactrum* | 1 (3.8) | 0 (0.0) | 1 (2.6) | 0 (0.0) |
| *anthropi* | | | | |
| *Proteus* Sp. | 4 (15.4) | 0 (0.0) | 5 (13.2) | 0 (0.0) |
| *Providencia rettgeri* | 0 (0.0) | 0 (0.0) | 1 (2.6) | 0 (0.0) |
| *Pseudomonas* | 4 (15.4) | 0 (0.0) | 4 (10.5) | 1 (5.6) |
| *aeruginosa* | | | | |
| *Serratia* | 2 (7.7) | 0 (0.0) | 2 (5.3) | 0 (0.0) |
| *marcescens* | | | | |
| Other gram- | 1 (3.8) | 1 (10.0) | 1 (2.6) | 1 (5.6) |
| negatives | | | | |
| **Other** | | | | |
| *Candida albicans* | 1 (3.8) | 0 (0.0) | 1 (2.6) | 0 (0.0) |

| | | | | |
|---|---|---|---|---|
| ^{a} modified Intent-to-Treat (mITT) Population = all patients randomized to receive gentamicin-sponge or any antimicrobial therapy who took any dose of gentamicin-sponge or systemic antibiotic therapy, and did not early-terminate from the study for failing to comply with the inclusion and exclusion criteria. | | | | |

Safety Population = all randomized patients who received any dose of gentamicin-sponge or systemic antibiotic therapy.

### Example 4 - Efficacy

At day 7 (the primary endpoint), 0 patients in the treatment group and 3 patients in the control group achieved clinical cure (*P* = 0.017). At days 10, 14, and 21, the control group had a non-significantly higher cumulative percent of patients with clinical cure compared to the treatment group. At the test-of-cure visit, however, the treatment group had a statistically significantly higher proportion of patients with clinical cure than the control group (22/26 [84.6%] versus 7/10 [70.0%]; P = 0.024).

When expressed as a proportion of the number of observed cases (i.e., counting only those patients who reached the final visit and whose clinical outcome was determinable), 22/22 (100.0%) patients in the treatment group achieved clinical cure compared with 7/10 (70%) in the control group. The treatment group also had a non-significantly higher cumulative percent of patients with clinical cure at day 28 than the control group (24/26 [92.3%] versus 7/10 [70.0%], *P* = 0.119). Using Kaplan-Meier estimates of time to clinical cure, 75% of patients achieved an outcome of clinical cure after 28 days in the treatment group compared with 40 days in the control group.

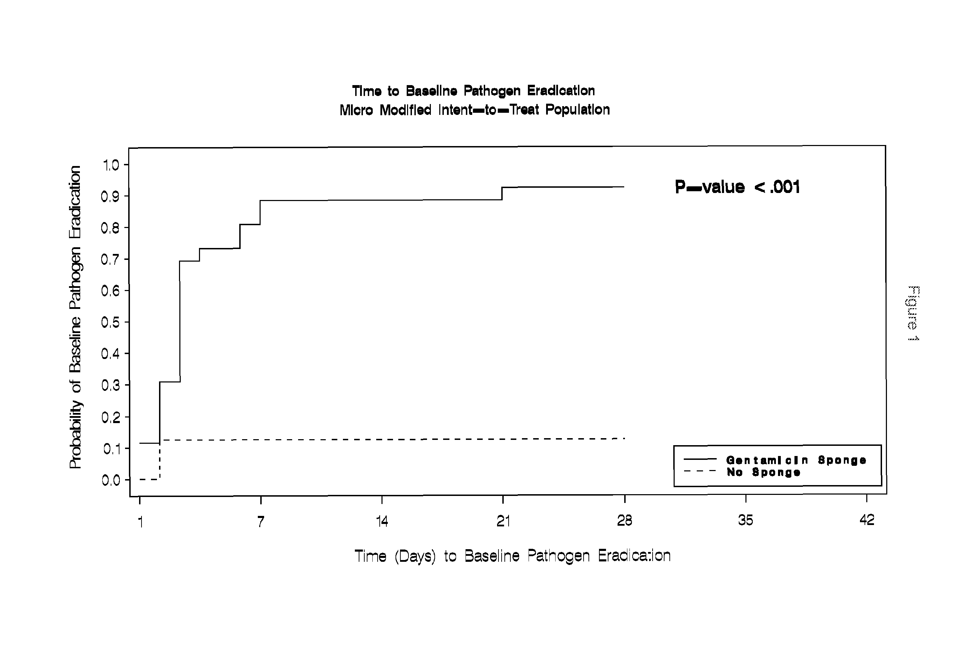

The proportion of patients with baseline pathogen eradication at day 3 was significantly higher in the treatment group than in the control group (20/26 [76.9%] versus 1/8 [12.5%]; *P* < 0.001 The pathogen eradication rate continued to be significantly higher in the treatment group throughout the remainder of the study (*P* ≤ 0.038 for each visit). At the test-of-cure visit, significantly more patients in the treatment group achieved baseline pathogen eradication than in the control group (24/26 versus 1/8; *P* < 0.001). Using Kaplan-Meier estimates, 75% of patients in the treatment group achieved baseline pathogen eradication 6 days after initiation of treatment; a comparative control group value could not be calculated because baseline pathogen eradication was observed for only 1 control patient during treatment. As shown in Figure 1, by 21 days after treatment the probability of achieving baseline pathogen eradication in the treatment group was 0.923 compared to 0.125 in the control group; the overall log-rank test comparing the likelihood of baseline pathogen eradication by treatment groups was statistically significant in favour of the treatment group (*P* < 0.001).

Although the study was not powered to detect statistically significant changes in Lipsky Wound Score or wound surface area from baseline, the difference in absolute reduction of the Lipsky Score at the final visit was significantly in favour of the treatment group (median -13 vs. -8, *P* = 0.042), although the difference in percent reduction was not statistically significant (median -77% vs. -64%, *P* = 0.376). However, there was no apparent trend suggesting either accelerated or delayed wound healing in the treatment group compared to the control group, based upon changes in wound surface area from baseline. Furthermore, there was no statistically significant difference in the percentage of patients who achieved complete wound closure at the end of treatment.

### Example 5 - Pharmacokinetics

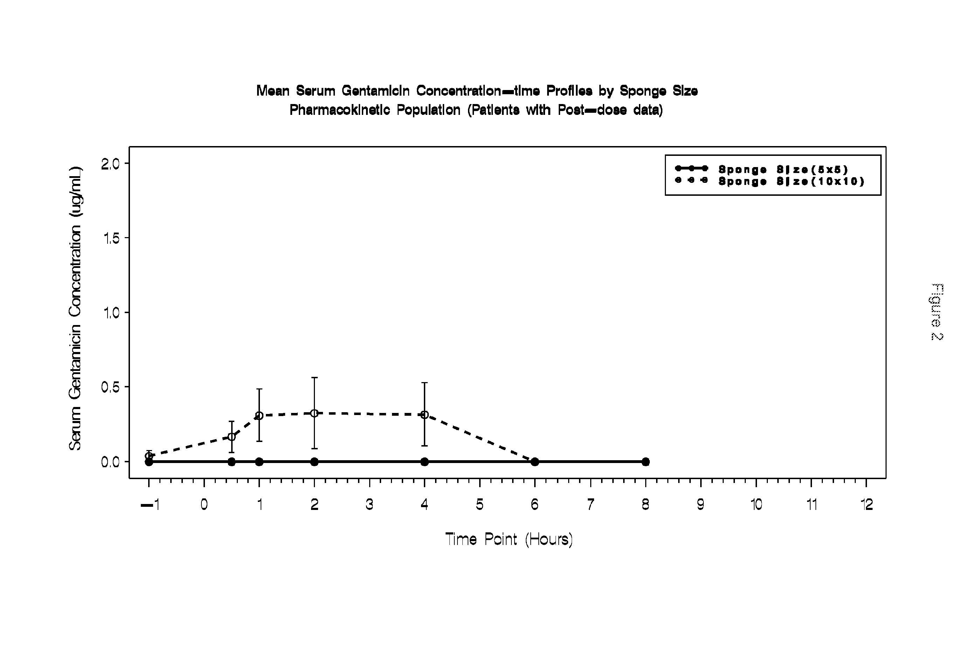

Referring to Figure 2, for all patients treated with the gentamicin-sponge at day 7, the trough serum gentamicin concentration was undetectable (i.e., ≤ 0.19 µg/mL, the lowest concentration that the analytical method can quantify). Post-dose serum gentamicin concentrations were quantifiable for 3 of the 5 patients who received the 10×10 cm sponge; the highest peak concentration recorded was 1.22 µg/mL at 2 hours post-dose for the patient who had the largest baseline wound area-- 39.3 cm². Post-dose concentrations were all undetectable for the 3 patients who received the 5×5 cm sponge.

### Example 6 - Safety

Of the 56 patients enrolled, 16 (28.6%) experienced one or more AEs during the study. The proportion of patients experiencing any AE was similar for the treatment (11/38 [28.9%]) and control (5/18 [27.8%]) groups. The most common AEs occurring in ≥2 patients per group were infected skin ulcer, tinea pedis, and increased blood creatinine. Other than increased blood creatinine, there were no clinically or statistically significant changes in laboratory tests or vital signs.

Most AEs were mild or moderate in severity but there were 6 SAEs; 5 in the treatment group (hypoglycemia, renal failure, cellulitis, tendon rupture, and wound hemorrhage), all of which resolved; and 1 in the control group (infected skin ulcer), which resolved with sequelae. Only one patient in each group experienced an AE that was considered definitely or probably related to the study drug: moderate renal failure in the treatment group (also reported as an SAE), and moderate drug intolerance in the control group. Both these related AEs resolved by the final visit. The SAE of tendon rupture reported in the treatment group was attributed to the concomitant oral levofloxacin therapy. No deaths occurred during the study and only one patient discontinued the study drug because of an AE.

### Conclusions

Using topical antimicrobial therapy for chronically infected wounds has great appeal. This study was designed to determine if adding the gentamicin-sponge to standard-of-care (systemic antibiotic therapy plus standard diabetic wound management) improves the rate of clinical cure of infection in diabetic patients with an infected foot ulcer of moderate severity.

This study was designed to use clinical cure at day 7 as a primary endpoint, and noted that the cure rate was significantly higher for the control group. The mean wound surface area at baseline, however, was nearly 4-fold greater, and the Lipsky Wound Scores were significantly higher, in patients randomized to the gentamicin-sponge compared to the control group. Although occurring by chance, this unfortunate imbalance in baseline wound severity may be attributable to the smaller than expected number of eligible patients and a lack of stratification by any baseline wound characteristic. These factors likely biased the results in favour of the control group, especially at the early visits. It is noteworthy that, despite this baseline imbalance and the relatively small sample size, the treatment group showed significantly higher rates of eradication of baseline pathogens from day 3 onwards and, most importantly, clinical cure at the test-of-cure visit. In fact, all treatment-group patients remaining in the study until the test-of-cure visit achieved clinical cure and we also observed a statistically significant greater reduction of Lipsky Wound Score from baseline at the final visit in the treatment group. Taken together, these findings suggest that when used in combination with systemic antibiotic therapy, the gentamicin-sponge may be more effective for treatment of DFIs than systemic therapy alone.

According to results of the susceptibility testing, a local concentration of up to 1000 µg/mL would exceed the gentamicin MICs for 92% (87 of 95) of the organisms isolated from the infected wounds at baseline. This likely accounts for the faster and more effective pathogen eradication observed with the gentamicin-sponge, and perhaps the superior clinical cure rate at the test-of-cure visit. Of note is that the pharmacokinetic results confirmed that patients had low serum gentamicin concentrations. Thus, patients should be able to derive the benefit of high local concentrations of gentamicin without being at risk for the potentially serious adverse effects of therapeutic serum concentrations.

By measuring the change in wound surface area from baseline, no apparent trend was found to suggest either accelerated or delayed wound healing in the treatment group compared to the control group. The study was not, however, adequately powered to detect such differences. The low incidence of drug-related AEs and analysis of all safety variables suggest that daily application of the gentamicin-sponge for up to 28 days appears to be safe and well tolerated.

The results of this study suggest that the gentamicin-sponge is safe and potentially beneficial when added to systemic antibiotic therapy for the treatment of diabetic foot infections of moderate severity.

The effect of an antibiotic can generally be classed as one of three mechanisms of action, typically based on the pharmacodynamic properties of the antibiotic - time-dependent effects, concentration dependent effects, and persistent effects. Aminoglycoside antibiotics are typically classed as exhibiting concentration-dependent effects, wherein the rate and/or extent of antibiotic effect is determined by the concentration of the antibiotic at the site of infection, and optimal effects are seen by maximal concentrations of antibiotic. However, maximal concentrations of this class of antibiotic often result in adverse reactions experienced by patients due to the normally high systemically-administered doses required to achieve optimal concentrations for antibiotic effect.

Without being bound by theory, it is thought that the present invention finds utility in treating bacterial infection by administration of at least one antibacterial agent, which antibacterial agent is administered remote from the site of infection; and by topical administration at the site of infection of a concentration-dependent antibiotic, such as aminoglycoside antibiotics; such that the concentration of aminoglycoside antibiotic reaches an optimal concentration for effect at the site of infection, without exposing the patient to the adverse reactions experienced, if such doses were administered systemically. In this connection, it is envisaged that the present invention is also useful in treating bacterial infection by administration of at least one antibacterial agent, which antibacterial agent is administered remote from the site of infection; and topical administration at the site of infection of at least one concentration-dependent antibiotic, for example, aminoglycoside antibiotics such as gentamicin, tobramycin, neomycin, amikacin, and netilmicin; lipopeptide molecules such as daptomycin; fluoroquinolones such as ciprofloxacin, moxifloxacin, and levofloxacin; and ketolides such as telithromycin.

## Claims

1. A composition comprising at least one aminoglycoside antibiotic for use in treating bacterial infection, comprising:
a. topical administration at the site of infection; and
b. administration of at least one antibacterial agent, which antibacterial agent is administered remote from the site of infection.

2. A method of treating bacterial infection at a site of infection, the method comprising the step of topically administering at least one aminoglycoside antibiotic at the site of infection; and administering at least one antibacterial agent, which at least one antibacterial agent is administered remote from the site of infection.

3. A composition for use according to Claim 1 or a method according to Claim 2, wherein the at least one aminoglycoside antibiotic is gentamicin, or a salt or prodrug thereof.

4. A composition for use according to Claim 1 or a method according to Claim 2, wherein the at least one aminoglycoside antibiotic is gentamicin sulphate.

5. A composition for use or a method according to Claim 4, wherein the gentamicin sulphate is administered in an equivalent amount of 32.5 - 130.0 mg of gentamicin.

6. A composition for use according to any one of Claims 1 and 3-5 or a method according to any one of Claims 2-5, wherein the at least one aminoglycoside antibiotic is administered topically as a composition comprising at least one aminoglycoside antibiotic and a polymeric matrix.

7. A method according to any one of Claims 2-6, wherein the topical administering step comprises the step of topically applying a composition comprising at least one aminoglycoside antibiotic and a polymeric matrix to the site of infection.

8. A composition for use according to Claim 6 or a method according to Claim 6 or 7, wherein the at least one aminoglycoside antibiotic is uniformly (or homogenously) dispersed throughout the polymeric matrix.

9. A composition for use according to Claim 6 or 8 or a method according to any one of Claims 6-8, wherein the polymeric matrix comprises collagen; optionally Type-I collagen.

10. A composition for use according to any one of Claims 6 and 8-9 or a method according to any one of Claims 6-9, wherein the at least one aminoglycoside antibiotic is uniformly (or homogenously) dispersed throughout the polymeric matrix in an amount of 0.1 - 10.0 mg/cm² of polymeric matrix; optionally in an amount of 1.0 - 5.5 mg/cm² of polymeric matrix; further optionally in an amount of 1.0 - 1.5 mg/cm² of polymeric matrix.

11. A composition for use or a method according to Claim 10, wherein the at least one aminoglycoside antibiotic is gentamicin, or a salt or prodrug thereof, and is uniformly (or homogenously) dispersed throughout the polymeric matrix in an amount equivalent to gentamicin of 1.3 mg/cm² of polymeric matrix.

12. A composition for use or a method according to Claim 10, wherein the at least one aminoglycoside antibiotic is gentamicin, or a salt or prodrug thereof, and is uniformly (or homogenously) dispersed throughout the polymeric matrix in an amount equivalent to gentamicin of 5.2 mg/cm² of polymeric matrix.

13. A composition for use according to any one of Claims 1, 3-6, and 8-12 or a method according to any one of Claims 2-12, wherein the at least one aminoglycoside antibiotic is gentamicin, or a salt or prodrug thereof, and is administered in an amount equivalent to gentamicin of 32.5 - 130.0 mg.

14. A composition for use according to any one of Claims 6, and 8-13 or a method according to any one of Claims 6-13, wherein the polymeric matrix has a two-dimensional surface area of 6.25 - 100 cm².

15. A composition for use according to any one of Claims 1, 3-6, and 8-14 or a method according to any one of Claims 2-14, wherein the at least one aminoglycoside antibiotic is gentamicin, or a salt or prodrug thereof, and is administered at least once daily in an amount equivalent to gentamicin of 32.5 - 130.0 mg; optionally is administered at least twice daily in an amount equivalent to gentamicin of 32.5 - 130.0 mg; further optionally is administered at least three times daily in an amount equivalent to gentamicin of 32.5 - 130.0 mg.

16. A composition for use according to any one of Claims 1, 3-6, and 8-15 or a method according to any one of Claims 2-15, wherein the at least one aminoglycoside antibiotic is gentamicin, or a salt or prodrug thereof, and is administered, optionally at regular intervals, at least once weekly in an amount equivalent to gentamicin of 32.5 - 130.0 mg; optionally is administered, optionally at regular intervals, between one and six times weekly in an amount equivalent to gentamicin of 32.5 - 130.0 mg.

17. A composition for use according to any one of Claims 1, 3-6, and 8-16 or a method according to any one of Claims 2-16, wherein the at least one antibacterial agent is administered by a route selected from oral and parenteral administration; optionally is administered by parenteral administration selected from intravenous and intramuscular administration.

18. A composition for use according to any one of Claims 1, 3-6, and 8-17 or a method according to any one of Claims 2-17, wherein the at least one antibacterial agent is selected from nitroimidazole compounds, lincosamides, sulfonamide compounds, dihydrofolate reductase inhibitors, lipopeptide molecules, tetracycline compounds, compounds comprising a beta-lactam moiety, glycopeptides, oxazolidinones, and quinolones.

19. A composition for use or a method according to Claim 18, wherein the at least one antibacterial agent is selected from fluoroquinolones.

20. A composition for use or a method according to Claim 19, wherein the fluoroquinolone is selected from ciprofloxacin, moxifloxacin, ofloxacin, balofloxacin, grepafloxacin, levofloxacin, pazufloxacin, sparfloxacin, temafloxacin, and tosufloxacin; optionally is selected from ciprofloxacin, moxifloxacin, and levofloxacin.

21. A composition for use or a method according to Claim 19 or 20, wherein the fluoroquinolone is levofloxacin.

22. A composition for use according to any one of Claims 1, 3-6, and 8-21 or a method according to any one of Claims 2-21, wherein the at least one aminoglycoside antibiotic is co-administered in combination with the at least one antibacterial agent.

23. A composition for use according to any one of Claims 1, 3-6, and 8-22 or a method according to any one of Claims 2-22, wherein the at least one antibacterial agent is administered prior to, and is co-administered in combination with the at least one aminoglycoside antibiotic.

24. A composition for use according to any one of Claims 1, 3-6, and 8-23 or a method according to any one of Claims 2-23, wherein the bacterial infection is an infection by a bacteria selected from at least one of an aerobic gram-negative bacteria, aerobic gram-positive bacteria, and anaerobic gram-negative bacteria.

25. A composition for use or a method according to Claim 24, wherein the gram-positive bacteria is selected from *Streptococcus, Staphylococcus, Enterococcus,* Gram positive cocci, and *Peptostreptococcus;* and the gram-negative bacteria is selected from *Acinetobacter, Alcaligenes, Bacteroides, Burkholderia, Enterobacter, Klebsiella, Morganella, Ochrobactrum, Proteus, Providencia, Pseudomonas,* and *Serratia.*

26. A composition comprising at least one aminoglycoside antibiotic for use in treating infected ulcers, optionally infected diabetic ulcers, comprising:
a. topical administration at the site of infection; and
b. administration of at least one antibacterial agent, which antibacterial agent is administered remote from the site of infection.

27. A method of treating infected ulcers, optionally infected diabetic ulcers, the method comprising the step of topically administering at least one aminoglycoside antibiotic at the site of infection; and administering at least one antibacterial agent, which at least one antibacterial agent is administered remote from the site of infection.

28. A composition for use according to Claim 26 or a method according to Claim 27, wherein the diabetic ulcer is a diabetic lower limb ulcer; optionally is a diabetic foot ulcer.
